# FASCICULE DE BREVET EUROPEEN

(11) **EP 2 146 991 B1**
(45) Date de publication et mention de la délivrance du brevet: **28.07.2010**
(21) Numéro de dépôt: 08787964.9
(22) Date de dépôt: 16.04.2008
(51) Int. Cl.: C07D 471/04, A61K 31/437, A61P 25/00, C07D 213/72, C07D 249/04, A61K 31/4353, A61P 35/00, A61P 19/00, A61P 31/00

(54) **DÉRIVÉS DE TRIAZOLOPYRIDINE-CARBOXAMIDES, LEUR PRÉPARATION ET LEUR APPLICATION EN THÉRAPEUTIQUE**
TRIAZOLOPYRIDIN-CARBOXAMID-DERIVATE SOWIE IHRE HERSTELLUNG UND THERAPEUTISCHE VERWENDUNG
TRIAZOLOPYRIDINE CARBOXAMIDE DERIVATIVES, PREPARATION THEREOF AND THERAPEUTIC USE THEREOF

(30) Priorité: 18.04.2007 FR 0702807
(43) Date de publication de la demande: 27.01.2010
(73) Titulaire: Sanofi-Aventis, 75013 Paris (FR)
(72) Inventeur: HOORNAERT, Christian, F-75013 Paris (FR)
(74) Mandataire: Gaslonde, Aude
(86) Numéro de dépôt international: PCT/FR2008/000535
(87) Numéro de publication internationale: WO 2008/145842

(56) Documents cités:
- WO-A-2006/138657

## Description

La présente invention se rapporte à des dérivés de triazolopyridine-carboxamides, à leur préparation et à leur application en thérapeutique.

La présente invention a pour objet les composés répondant à la formule (I) 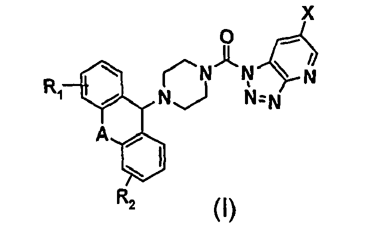 dans laquelle :
X représente un atome d'hydrogène, un atome d'halogène, un groupe (C₁-C₆)alkyle, (C₁-C₆)alcoxy, halo(C₁-C₆)alkyle, un groupe S(O)ₘR", hydroxy ou cyano,
A est absent ou bien représente une liaison, un atome d'oxygène, un atome de soufre, un groupe NR, C(O)NR', SO₂NR', un groupe (C₁-C₂)alkylène, ou un groupe (C₂)alcényle,
R₁ et R₂ représentent indépendamment l'un de l'autre, un ou plusieurs groupes choisis parmi un atome d'hydrogène, un groupe (C₁-C₆)alkyle, un groupe (C₁-C₆)alcoxy, (C₃-C₇)cycloalkyle, (C₃-C₇)cycloalkyle(C₁-C₆)alkyle, un groupe (C₃-C₇)cycloalkyle(C₁-C₆)alcoxy, un atome d'halogène, un groupe cyano, C(O)R', C(O)OR', C(O)NR₁₀R₂₀, NO₂, un groupe NR₁₀R₂₀, NR₁₀C(O)-R₂₀, les groupes (C₁-C₆)alkyle et (C₁-C₆)alcoxy étant éventuellement substitués par un ou plusieurs atomes ou groupes choisis indépendamment les uns des autres parmi les atomes d'halogène, les groupes hydroxy, amino, NR₁₀R₂₀,
R représente un groupe choisi parmi un atome d'hydrogène, un groupe (C₁-C₆)alkyle, (C₃-C₇)cycloalkyle, (C₃-C₇)cycloalkyle(C₁-C₆)alkyle, C(O)R', SO₂R", CO₂R", C(O)NR₁₀R₂₀,
R' représente un groupe choisi parmi un atome d'hydrogène, un groupe (C₁-C₆)alkyle, (C₃-C₇)cycloalkyle, (C₃₋C₇)cycloalkyle(C₁-C₆)alkyle,
R" représente un groupe choisi parmi un groupe (C₁-C₆)alkyle, (C₃-C₇)cycloalkyle, (C₃-C₇)cycloalkyle(C₁-C₆)alkyle,
R₁₀ et R₂₀ représentent, indépendamment l'un de l'autre, un ou plusieurs groupes choisis parmi un atome d'hydrogène, un groupe (C₁-C₆)alkyle, (C₃-C₇)cycloalkyle, (C₃-C₇)cycloalkyle(C₁-C₆)alkyle ou bien R₁₀ et R₂₀ peuvent former un cycle saturé ou partiellement insaturé, contenant de 5 à 7 atomes de carbone et contenant éventuellement un hétéroatome choisi parmi O, N ou S(O)ₘ,
m représente 0, 1 ou 2.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvates, à savoir sous forme d'associations ou de combinaisons avec une ou plusieurs molécules d'eau ou avec un solvant. De tels hydrates et solvates font également partie de l'invention.

Dans le cadre de la présente invention, on entend par :
- C_{t-z} où t et z peuvent prendre les valeurs de 1 à 7, une chaîne ou un cycle carboné pouvant avoir de t à z atomes de carbone, par exemple C₁₋₃ peut caractériser une chaîne carbonée ayant de 1 à 3 atomes de carbone;
- un atome d'halogène : un fluor, un chlore, un brome ou un iode ;
- un groupe alkyle : un groupe aliphatique saturé linéaire ou ramifié. A titre d'exemples, on peut citer les groupes méthyle, éthyle, propyle, isopropyle, butyle, isobutyle, tertbutyle, pentyle, etc ;
- un groupe cycloalkyle : un groupe aliphatique cyclique saturé. A titre d'exemples, on peut citer les groupes cyclopropyle, cyclobutyle, cyclopentyle, cyclohexyle, etc ;
- un groupe alkylène : un groupe aliphatique divalent saturé, linéaire ou ramifié.
   A titre d'exemple un groupe C₁₋₃-alkylène représente une chaîne carbonée divalente de 1 à 3 atomes de carbone, linéaire ou ramifiée, telle que un méthylènyle (-CH₂-), un éthylènyle (-CH₂CH₂-), un 1-méthyléthylènyle (-CH(CH₃)CH₂-), un propylènyle (-CH₂CH₂CH₂-), etc ;
- un groupe alcényle : un groupe aliphatique mono- ou poly-insaturé, linéaire ou ramifié, comprenant par exemple une ou deux insaturations éthyléniques ; à titre d'exemple, un groupe (C₂)alcényle représente une chaîne carbonée contenant 2 atomes de carbone et une insaturation éthylénique, tel qu'un éthényle (-CH=CH-) ;
- un groupe alcoxy : un radical -O-alkyle où le groupe alkyle est tel que précédemment défini ;
- un groupe haloalkyle : un groupe alkyle dont un ou plusieurs atomes d'hydrogène ont été substitués par un atome d'halogène. A titre d'exemples, on peut citer les groupes -CF₃, -CH₂CF₃ ;
- les atomes de soufre peuvent être présents à l'état oxydé (sulfoxide, sulfone).

Dans les différents groupes tel que définis ci-dessous, les groupes R₁, R₂, R et R', lorsqu'ils ne sont pas définis, ont les mêmes définitions que celles mentionnées ci-dessus.

Parmi les composés de formule (I) objets de l'invention, un premier groupe de composés est constitué par les composés pour lesquels :
X représente un atome d'hydrogène ou d'halogène,
A est absent ou bien représente une liaison, un atome d'oxygène, un atome de soufre, un groupe NR, C(O)NR', SO₂NR', un groupe (C₁-C₂)alkylène, ou un groupe (C₂)alcényle.

Parmi les composés de formule (I) objets de l'invention, un second groupe de composés est constitué par les composés de formule (I) pour lesquels :
X représente un atome d'hydrogène ou d'halogène,
A est absent ou bien représente une liaison ou un groupe (C₁-C₂)alkylène,
R₁ et R₂ représentent indépendamment l'un de l'autre, un ou plusieurs groupes choisis parmi un atome d'hydrogène, un atome d'halogène, un groupe cyano ou alcoxy.

Parmi les composés de formule (I) objets de l'invention, un troisième groupe de composés est constitué par les composés de formule (I) pour lesquels :
X représente un atome d'hydrogène ou d'halogène,
A est absent ou bien représente une liaison ou un groupe éthylène,
R₁ et R₂ représentent indépendamment l'un de l'autre un ou plusieurs groupes choisis parmi un atome d'hydrogène ou d'halogène.

Parmi les composés de formule (I) objets de l'invention, un quatrième groupe de composés est constitué par les composés de formule (I) pour lesquels:
X représente un atome d'hydrogène ou de chlore,
A est absent ou bien représente une liaison ou un groupe éthylène,
R₁ et R₂ représentent indépendamment l'un de l'autre un ou plusieurs groupes choisis parmi un atome d'hydrogène, un atome de fluor ou un atome de chlore.

Les combinaisons des groupes un à quatre tels que définis ci dessus font également partie de l'invention.

Parmi les composés de formule (I) objets de l'invention, on peut notamment citer les composés suivants :
- (4-benzhydryl-pipérazin-1-yl)-[1,2,3]triazolo[4,5-b]pyridin-1-yl-méthanone ;
- (4-benzhydryl-pipérazin-1-yl)-(6-chloro-[1,2,3]triazolo[4,5-b]pyridin-1-yl)-méthanone ;
- {4-[bis-(4-fluoro-phényl)-méthyl]-pipérazin-1-yl}-[1,2,3]triazolo[4,5-b]pyridin-1-yl-méthanone ;
- {4-[bis-(4-chloro-phényl)-méthyl]-pipérazin-1-yl}-[1,2,3]triazolo[4,5-b]pyridin-1-yl-méthanone ;
- {4-[bis-(4-fluoro-phényl)-méthyl]-pipérazin-1-yl}-(6-chloro-[1,2,3]triazolo[4,5-b]pyridin-1-yl)-méthanone ;
- {4-[bis-(4-chloro-phényl)-méthyl]-pipérazin-1-yl}-(6-chloro-[1,2,3]triazolo[4,5-b]pyridin-1-yl)-méthanone;
- [4-(10,11-dihydro-5H-dibenzo[a,d]cycloheptèn-5-yl)-pipérazin-1-yl]-[1,2,3]triazolo[4,5-b]pyridin-1-yl-méthanone ; .
- [4-(9H-fluorèn-9-yl)-pipérazin-1-yl]-[1,2,3]triazolo[4,5-b]pyridin-1-yl-méthanone.

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 2nd Edition (John Wiley & Sons, Inc., New York), 1991.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 3rd Edition, Wiley Interscience, 1985, p. 310-316.

Conformément à l'invention, on peut préparer les composés de formule générale (I) selon le procédé qui suit.

Une première méthode (schéma 1) consiste à faire réagir un dérivé de triazolopyridine de formule générale (II) dans laquelle X est tel que défini ci-dessus, avec un chlorure de carbamoyle de formule générale (III) dans laquelle R₁, R₂ et A sont tels que définis ci-dessus, dans un solvant tel que le tétrahydrofurane, en présence d'une base telle que la diisopropyléthylamine et d'un catalyseur tel que la 4-diméthylaminopyridine.

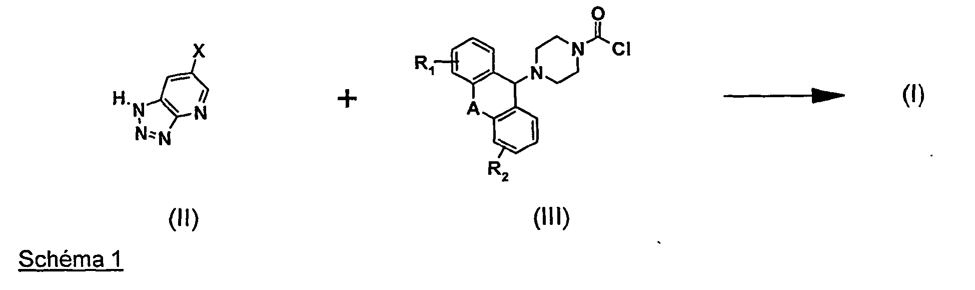

Une seconde méthode (schéma 2) consiste à faire réagir un dérivé de triazolopyridine de formule générale (IV) dans laquelle X est tel que défini ci-dessus, avec un dérivé de formule générale (V) dans laquelle R₁, R₂ et A sont tels que définis ci-dessus et L représente un groupe partant, dans un solvant tel que l'acétonitrile et en présence d'une base telle que la diisopropyléthylamine.

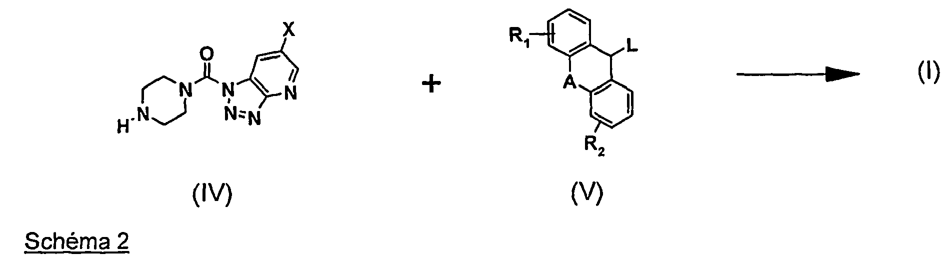

Les composés de formule générale (II), (III), (IV) et (V), quand leur mode de préparation n'est pas décrit ci-dessus, sont disponibles dans le commerce ou décrits dans la littérature, ou bien peuvent être préparés selon des méthodes qui y sont décrites ou qui sont connues de l'Homme du métier.

L'invention, selon un autre de ses aspects, a également pour objet les composés de formule (IV), dans laquelle X est tel que défini ci-dessus, sous leur forme de base ou de sels. Ces composés sont utiles comme intermédiaires de synthèse des composés de formule (I).

Les exemples qui suivent illustrent la préparation de quelques composés de l'invention. Ces exemples ne sont pas limitatifs et ne font qu'illustrer l'invention. Les microanalyses, les spectres IR et RMN et /ou les analyses LC-MS confirment les structures et les puretés des composés obtenus. Les numéros des composés exemplifiés renvoient à ceux donnés dans le tableau ci-après, qui illustre les structures chimiques et les propriétés physiques de quelques composés selon l'invention.

### Exemple 1

### (4-benzhydryl-pipérazin-1-yl)-[1,2,3]triazolo[4,5-b]pyridin-1-yl-méthanone

### 1.1. Chlorure de 4-benzhydryl-pipérazine-1-carbonyle

A une solution de 1,187 g (4 mmoles) de trisphogène dans 10 mL de dichlorométhane, refroidie à -5°C sous atmosphère d'Argon, on ajoute goutte à goutte une solution de 2,522 g (10 mmoles) de 1-benzhydryl-piperazine et de 1,62 mL (20 mmoles) de pyridine dans 15 mL de dichlorométhane. On poursuit l'agitation à -5°C pendant 15 minutes puis à température ambiante pendant 3 heures. On ajoute ensuite 50 mL de dichlorométhane et 50 mL d'eau. On décante et on lave la phase organique par 2x25 mL d'eau puis 25 mL d'une solution aqueuse saturée en chlorure de sodium. On sèche sur sulfate de sodium et on évapore sous vide pour obtenir 2,72 g de produit sous forme de gomme, utilisé tel quel dans l'étape suivante.

### 1.2. (4-benzhydryl-pipérazin-1-yl)-[1,2,3]triazolo[4,5-b]pyridin-1-yl-méthanone

A une suspension de 0,865 g (2,75 mmoles) de chlorure de 4-benzhydryl-pipérazine-1-carbonyle obtenu à l'étape 1.1., de 0,300 g (2,50 mmoles) de 1*H-*[1,2,3]triazolo[4,5-*b*]pyridine et de 0,015 g (0,12 mmole) de 4-diméthylaminopyridine dans 5 mL de tétrahydrofurane, on ajoute 0,62 mL (3,75 mmoles) de diisopropyléthylamine. On agite à température ambiante pendant 4 heures puis on ajoute 60 mL d'acétate d'éthyle et 15 mL d'eau. On décante la phase organique et on la lave par 2x15 mL d'eau puis 15 mL d'une solution aqueuse saturée en chlorure de sodium. On sèche sur sulfate de sodium et on évapore sous vide. On purifie le produit par chromatographie sur gel de silice en éluant par un mélange 25:75 puis 30:70, 35:65 et 40:60 d'acétate d'éthyle et de cyclohexane. On recristallise ensuite dans l'isopropanol pour obtenir 0,63 g (1,58 mmole) de produit sous forme de cristaux blancs.
Point de fusion (°C) : 146-148 (décomposition)
LC-MS (m/z) : 399 (MH+)
IR (KBr, cm⁻¹) : 1697
¹H-RMN (CDCl₃, δ ppm) : 8,80 (dd, 1H), 8,40 (dd, 1H), 7,55 (dd, 1H), 7,45-7,15 (m, 10H), 4,35 (s, 1 H), 4,00 (m, 4H), 2,60 (m, 4H).

### Exemple 2

### (4-benzhydryl-pipérazin-1-yl)-(6-chloro-[1,2,3]triazolo[4,5-b]pyridin-1-yl)-méthanone

On procède comme décrit à l'exemple 1 en utilisant 0,651 g (2,07 mmoles) de chlorure de 4-benzhydryl-pipérazine-1-carbonyle et 0,278 g (1,80 mmole) de 6-chloro-1 H-[1,2,3]triazolo[4,5-b]pyridine (J. Am. Chem. Soc. 1949, 1885). On recristallise le produit dans un mélange de 2-butanone et de diisopropyléther pour obtenir 0,46 g (1,06 mmole) de produit sous forme de cristaux blancs.
Point de fusion (°C) : 173-175 (décomposition)
LC-MS (m/z) : 455 (MNa+), 471 (MK+)
IR (KBr, cm⁻¹) : 1706
¹H-RMN (CDCl₃, δ ppm) : 8,75 (s, 1H), 8,30 (s, 1H), 7,45 (m, 4H), 7,35-7,20 (m, 6H), 4,35 (s, 1H), 4,00 (m, 4H), 2,65 (m, 4H).

### Exemple 3

### {4-[bis-(4-fluoro-phényl)-méthyl]-pipérazin-1-yl}-[1,2,3]triazolo[4,5-b]pyridin-1-yl-méthanone

On procède suivant l'exemple 1 en utilisant 0,757 g (2,16 mmoles) de chlorure de 4-[bis-(4-fluoro-phényl)-méthyl]-pipérazine-1-carbonyle (obtenu suivant l'exemple 1.1) et 0,216 g (1,80 mmole) de 1H-[1,2,3]triazolo[4,5-b]pyridine. On recristallise dans l'isopropanol pour obtenir 0,50 g (1,15 mmole) de produit sous forme de cristaux blancs.
Point de fusion (°C) : 151-153 (décomposition)
LC-MS (m/z) : 435 (MH+)
IR (KBr, cm⁻¹) : 1712
¹H-RMN (CDCl₃, δ ppm) : 8 ,85 (d, 1 H), 8,40 (d, 1 H), 7,55 (dd, 1 H), 7,40 (m, 4H), 7,05 (m, 4H), 4,40 (s, 1 H), 4,00 (m, 4H), 2,60 (m, 4H).

### Exemple 4

### {4-[bis-(4-chloro-phényl)-méthyl]-pipérazin-1-yl}-[1,2,3]triazolo[4,5-b]pyridin-1-yl-méthanone

On procède suivant l'exemple 1 en utilisant 0,882 g (2,30 mmoles) de chlorure de 4-[bis-(4-chloro-phényl)-méthyl]-pipérazine-1-carbonyle (obtenu suivant l'exemple 1.1) et 0,240 g (2 mmoles) de 1H-[1,2,3]triazolo[4,5-b]pyridine. On recristallise dans un mélange de 2-butanone et de diisopropyléther pour obtenir 0,57 g (1,22 mmole) de produit sous forme de cristaux blancs.
Point de fusion (°C) : 163-165 (décomposition)
LC-MS (m/z) : 467 (MH+)
IR (KBr, cm⁻¹) : 1715
¹H-RMN (CDCl₃, δ ppm) : 8,70 (d, 1H), 8,30 (d, 1H), 7,45 (dd, 1H), 7,30-7,15 (m, 8H), 4,25 (s, 1 H), 3,90 (m, 4H), 2,50 (m, 4H).

### Exemple 5

### {4-[bis-(4-fluoro-phényl)-méthyl]-pipérazin-1-yl}-(6-chloro-[1,2,3]triazolo[4,5-b]pyridin-1-yl)-méthanone

On procède suivant l'exemple 1 en utilisant 0,757 g (2,16 mmoles) de chlorure de 4-[bis-(4-fluoro-phényl)-méthyl]-pipérazine-1-carbonyle et 0,278 g (1,80 mmole) de 6-chloro-1H-[1,2,3]triazolo[4,5-b]pyridine. On recristallise dans l'isopropanol pour obtenir 0,60 g (1,28 mmole) de produit sous forme de cristaux blancs.
Point de fusion (°C) : 167-169 (décomposition)
LC-MS (m/z) : 469 (MH+)
IR (KBr, cm⁻¹) : 1704
¹H-RMN (CDCl₃, δ ppm) : 8,75 (s, 1H), 8,40 (s, 1H), 7,40 (m, 4H), 7,05 (m, 4H), 4,35 (s, 1 H), 4,00 (m, 4H), 2,60 (m, 4H).

### Exemple 6

### {4-[bis-(4-chloro-phényl)-méthyl]-pipérazin-1-yl}-(6-chloro-[1,2,3]triazolo[4,5-b]pyridin-1-yl)-méthanone

On procède suivant l'exemple 1 en utilisant 0,926 g (2,41 mmoles) de chlorure de 4-[bis-(4-chloro-phényl)-méthyl]-pipérazine-1-carbonyle et 0,324 g (2,10 mmoles) de 6-chloro-1*H*-[1,2,3]triazolo[4,5-b]pyridine. On recristallise dans un mélange de 2-butanone et de diisopropyléther pour obtenir 0,71 g (1,41 mmole) de produit sous forme de cristaux blancs.
Point de fusion (°C) : 172-174 (décomposition)
IR (KBr, cm⁻¹) : 1702
¹H-RMN (d₆-DMSO, δ ppm) : 8,85 (s, 1H), 8,50 (s, 1H), 7,45 (d, 4H), 7,40 (d, 4H), 4,55 (s, 1H), 3,80 (m, 4H), 2,45 (m, 4H).

### Exemple 7

### [4-(10,11-dihydro-5H-dibenzo[a,d]cycloheptèn-5-yl)-pipérazin-1-yl]-[1,2,3]triazolo[4,5-b]pyridin-1-yl-méthanone

On procède suivant l'exemple 1 en utilisant 0,790 g (2,32 mmoles) de chlorure de 4-(10,11-dihydro-5H-dibenzo[a,d]cycloheptèn-5-yl)-pipérazine-1-carbonyle (obtenu suivant l'exemple 1.1) et 0,278 g (2,32 mmoles) de 1*H*-[1,2,3]triazolo[4,5-b]pyridine. On recristallise dans l'acétate d'éthyle pour obtenir 0,26 g (0,61 mmole) de produit sous forme de cristaux blancs.
Point de fusion (°C) : 190-194 (décomposition)
LC-MS (m/z) : 447 (MNa+), 463 (MK+), 871 (MMNa+)
IR (KBr, cm⁻¹) : 1707
¹H-RMN (CDCl₃, δ ppm) : 8,80 (d, 1H), 8,35 (d, 1H), 7,55 (dd, 1H), 7,25-7,05 (m, 8H), 4,15-3,85 (m, 7H), 2,95-2,80 (m, 2H), 2,55 (m, 4H).

### Exemple 8

### [4-(9H-Fluorèn-9-yl)-pipérazin-1-yl]-[1,2,3]triazolo[4,5-b]pyridin-1-yl-méthanone

### 8.1. 4-([1,2,3]triazolo[4,5-b]pyridine-1-carbonyl)-pipérazine-1-carboxylate de tert-butyle

A une suspension de 0,840 g (7 mmoles) de 1*H*-[1,2,3]triazolo[4,5-b]pyridine, de 1,741 g (7 mmoles) de 4-chlorocarbonyl-pipérazine-1-carboxylate de *tert*-butyle (Bioorg. Med. Chem. Lett. 2000, 10, 2357) et de 0,042 g (0,35 mmole) de 4-diméthylaminopyridine dans 14 mL de tetrahydrofurane, on ajoute 1,39 mL (8,40 mmoles) de diisopropyléthylamine. On agite 4 heures à température ambiante puis on ajoute 80 mL d'acétate d'éthyle et 20 mL d'eau. On décante la phase organique puis on la lave par 3x20 mL d'eau et 20 mL d'une solution aqueuse saturée en chlorure de sodium. On sèche sur sulfate de sodium et on évapore à sec. On recristallise le résidu dans l'isopropanol pour obtenir 1,70 g (5,1 mmoles) de produit sous forme de cristaux blancs.
Point de fusion (°C) : 155-157 (décomposition)

### 8.2. Dichlorhydrate de pipérazin-1-yl-[1,2,3]triazolo[4,5-b]pyridin-1-yl-méthanone

A une solution de 0,498 g (1,50 mmole) de 4-([1,2,3]triazolo[4,5-b]pyridine-1-carbonyl)-pipérazine-1-carboxylate de *tert*-butyle préparé à l'étape 8.1., dans 7,5 mL de dichlorométhane on ajoute 1,8 mL d'une solution 5N d'acide chlorhydrique (9 mmoles) dans l'isopropanol. On poursuit l'agitation pendant une nuit à température ambiante. On filtre le solide formé, on le lave par 3 mL de dichlorométhane et par 2x6 mL de diisopropyléther puis on le sèche sous vide en présence de pentoxyde de phosphore pour obtenir 0,46 g (1,5 mmole) de produit sous forme de poudre blanche.
Point de fusion (°C) : 160 (décomposition)
LC-MS (m/z) : 233 (MH+)
IR (KBr, cm⁻¹) : 1714
¹H-RMN (d₆-DMSO, δ ppm) : 9,60 (m, 2H), 8,80 (d, 1H), 8,40 (d, 1H), 7,75 (dd, 1H), 4,05 (m, 4H), 3,25 (m, 4H)

### 8.3. [4-(9H-fluorèn-9-yl)-pipérazin-1-yl]-[1,2,3]triazolo[4,5-b]pyridin-1-yl-méthanone

A une suspension de 0,381 g (1,25 mmole) de dichlorhydrate de pipérazin-1-yl-[1,2,3]triazolo[4,5-b]pyridin-1-yl-méthanone, préparé à l'étape 8.2., et de 0,337 g (1,38 mmole) de 9-bromo-9*H*-fluorene dans 4 mL d'acétonitrile, on ajoute 0,83 mL (5 mmoles) de diisopropyléthylamine. On agite la solution une nuit à température ambiante puis on évapore. On reprend le résidu dans un mélange de 40 mL d'acétate d'éthyle et de 10 mL d'eau. On décante et on lave la phase organique par 2x10 mL d'eau puis par 10 mL d'une solution aqueuse saturée en chlorure de sodium. On sèche sur sulfate de sodium et on évapore sous vide. On purifie le produit par chromatographie sur gel de silice en éluant par un mélange 40:60 puis 50:50 d'acétate d'éthyle et de cyclohexane puis on recristallise dans l'isopropanol pour obtenir 0,40 g (1,01 mole) de produit sous forme de cristaux blancs.
Point de fusion (°C) : 170-172 (décomposition)
LC-MS (m/z) : 397 (MH+), 815 (MMNa+)
IR (KBr, cm⁻¹) : 1723
¹H-RMN (CDCl₃, δ ppm) : 8,80 (dd, 1H), 8,35 (dd, 1H), 7,75-7,65 (m, 4H), 7,55 (dd, 1H), 7,45-7,25 (m, 4H), 4,95 (s, 1H), 3,95 (m, 4H), 2,85 (m, 4H).

Le tableau qui suit illustre les structures chimiques et les propriétés physiques de quelques exemples de composés selon l'invention. Dans ce tableau :
- PF (°C) représente le point de fusion du composé en degrés Celsius ;
- dans la colonne « A », « - » signifie que A est absent ;
- dans la colonne « sel », « - » représente un composé sous forme de base libre, alors que « HCl » représente un composé sous forme de chlorhydrate.

Les composés décrits dans ce tableau ont été préparés selon les méthodes décrites précédemment.

**Tableau 1**

| | | | | | | |
|---|---|---|---|---|---|---|
| 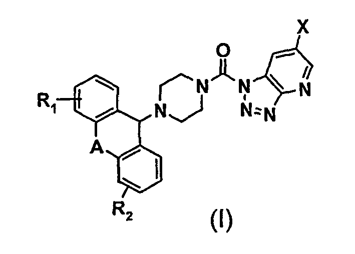 | | | | | | |

| **N°** | **X** | **A** | **R1** | **R2** | **Sel** | **PF (°C)** |
|---|---|---|---|---|---|---|
| **1** | H | - | H | H | - | 146-148 |
| **2** | Cl | - | H | H | - | 173-175 |
| **3** | H | - | 4-F | 4-F | - | 151-153 |
| **4** | H | - | 4-Cl | 4-Cl | - | 163-165 |
| **5** | Cl | - | 4-F | 4-F | - | 167-169 |
| **6** | Cl | - | 4-CI | 4-Cl | - | 172-174 |
| **7** | H | -CH₂CH₂- | H | H | - | 190-194 |
| **8** | H | liaison | H | H | - | 170-172 |

Les composés selon l'invention présentent de façon surprenante un effet inhibiteur sur l'enzyme MGL (monoacyl glycerol lipase). L'enzyme MGL catalyse l'hydrolyse de dérivés endogènes d'esters monoglycérides de différents acides gras (FEBS Letters 1998, 429, 152-156) et en particulier l'hydrolyse du 2-arachidonoylglycérol (2-AG) et du 1 (3)-arachidonoylglycérol (1(3)-AG) (J. Biol. Chem. 1987, 272 (48), 27218-27223; Proc. Natl. Acad. Sci. USA 2002, 99 (16), 10819-10824 ; Biochem. Pharmacol. 2004, 67, 1381-1387 ; Mol. Pharmacol. 2004, 66 (5), 1260-1264). Les dérivés 2-AG et 1-(3)-AG en particulier interagissent avec les récepteurs cannabinoïdes (J. Biol. Chem. 1999, 274 (5), 2794-2801 ; J. Biol. Chem. 2000, 275 (1), 605-612 ; British. J. Pharmacol. 2001, 134, 664-672).

Les composés de l'invention bloquent cette voie de dégradation et augmentent les taux tissulaires de ces dérivés et en particulier du 2-AG et/ou du 1 (3)-AG. A ce titre, ils peuvent être utilisés dans la prévention et le traitement de pathologies dans lesquelles sont impliqués le 2-AG et/ou le 1 (3)-AG en particulier et/ou tout autre substrat métabolisé par l'enzyme MGL (Progress Lipid Research 2006, 45, 405-446).

Les composés selon l'invention ont fait l'objet d'essais pharmacologiques permettant de déterminer leur effet inhibiteur de l'enzyme MGL.

Des essais ont consisté à mesurer l'activité *in vitro* des composés de l'invention sur l'enzyme MGL.

L'activité inhibitrice vis à vis de MGL est donnée par la concentration qui inhibe 50% de l'activité de MGL.

L'activité inhibitrice a été mesurée dans un test radio-enzymatique basé sur la mesure du produit d'hydrolyse du 2-Oléoyl Glycérol ([³H] 2-OG) par la MGL. Les produits d'hydrolyse du [³H] 2-OG, marqué sur le glycérol, sont l'acide oléïque et le [³H]glycérol et la source d'enzyme MGL est un homogénat de cerveau de souris où le cervelet et le bulbe rachidien ont été éliminés. Les cerveaux de souris sont prélevés, stockés à -80°C jusqu'à leur utilisation ou homogénéisés immédiatement 2 fois 5 secondes à l'aide de l'appareil Precellys à 5000 rpm (Bertin) dans un tampon Tris-HCl 10 mM, NaCl 150mM, EDTA 1mM (pH8) à 4°C. La concentration des homogénats est ensuite ajustée à 7,5 µg/µL.

La gamme de dilution des composés est réalisée à partir de solutions stocks à 20 mM en 100 % DMSO. La première dilution de cette gamme est réalisée en 100 % DMSO puis la deuxième dans le tampon de réaction enzymatique (50 mM phosphate, BSA 0,1 %) aboutissant à la réalisation d'une gamme de concentration 10 fois concentrée. Les composés à tester sont préincubés à la concentration choisie pendant 20 minutes avec la préparation d'homogénat de cerveaux de souris. La concentration finale de DMSO dans la réaction enzymatique n'excède pas 0,1%.

Le dosage de l'activité MGL est réalisée en microplaque 96 puits dans un volume réactionnel final de 100 µL. Brièvement, 75 µg de protéines, préincubés avec les composés à tester, sont dilués dans 50 mM de tampon phosphate contenant 0,1 % de BSA et incubés, pendant 20 minutes à température ambiante, en présence de 50 µM de 2-OG contenant une quantité de [³H] 2-OG de 0,027 µCi/puits (Activité spécifique de 20 Ci/mmole). La réaction est arrêtée et les produits formés sont séparés par l'addition et le mélange de 100 µL de chloroforme/méthanol (1/1). Après 10 minutes d'agitation, la microplaque est centrifugée pendant 15 minutes à 4000g et un aliquot de 30 µL de la phase aqueuse contenant le [³H]glycérol produit est prélevé puis compté pendant 5 minutes par scintillation liquide (Wallac 1450 Microbeta).

Dans ces conditions, les composés les plus actifs de l'invention présentent une Cl₅₀ (concentration inhibant de 50% l'activité enzymatique contrôle de la MGL) comprise entre 0,001 et 0,1 µM.

Par exemple, les composés n° 1 et 7 ont montré une Cl₅₀ de respectivement 0,004 et 0,025 µM.

Il apparaît donc que les composés selon l'invention ont une activité inhibitrice vis-à-vis de MGL.

Les composés selon l'invention peuvent donc être utilisés pour la préparation de médicaments, en particulier de médicaments inhibiteurs de l'enzyme MGL.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvate du composé de formule (I).

Ces médicaments trouvent leur emploi en thérapeutique, notamment dans le traitement et la prévention de:
la douleur notamment les douleurs aiguës ou chroniques de type neurogène : migraine, douleurs neuropathiques incluant les formes associées au virus de l'herpès et au diabète ;
les douleurs aiguës ou chroniques associées aux maladies inflammatoires: arthrite, arthrite rhumatoïde, ostéoarthrite, spondylite, goutte, vascularite, maladie de Crohn, syndrome du colon irritable ;
les douleurs aiguës ou chroniques périphériques ;
les vertiges, les vomissements, les nausées en particulier celles consécutives à une chimiothérapie ;
les troubles du comportement alimentaire en particulier les anorexies et cachexies de diverses natures ;
le syndrome métabolique et ses manifestations, incluant l'obésité ;
les dyslipidémies et leurs manifestations, incluant l'athérosclérose et les maladies coronariennes ;
les pathologies neurologiques et psychiatriques : tremblements, dyskinésies, dystonies, spasticité, comportements compulsifs et obsessionnels, syndrome de Tourette, toutes les formes de dépression et d'anxiété de toute nature et origine, troubles de l'humeur, psychoses ;
les maladies neuro-dégénératives aiguës et chroniques : maladie de Parkinson, maladie d'Alzheimer, démence sénile, chorée de Huntington, lésions liées à l'ischémie cérébrale et aux traumatismes crâniens et médullaires, la sclérose latérale amyotrophique ;
l'épilepsie ;
les troubles du sommeil incluant les apnées du sommeil ;
les maladies cardiovasculaires en particulier hypertension, arythmies cardiaques, artériosclérose, crise cardiaque, ischémies cardiaques ;
l'ischémie rénale ;
les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaires, astrocytomes, astroblastomes, épendyomes, oligodendrogliomes, tumeur du plexus, neuroepithéliomes, tumeur de l'épiphyse, épendymoblastomes, méningiomes malins, sarcomatoses, mélanomes malins, schwénnomes) ;
les désordres du système immunitaire, notamment les maladies auto-immunes : psoriasis, lupus érythémateux, maladies du tissu conjonctif ou connectivites, syndrome de Sjögren's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire ;
les maladies allergiques : l'hypersensibilité immédiate ou retardée, rhinites ou conjonctivites allergiques, dermatites de contact ;
les maladies infectieuses parasitaires, virales ou bactériennes : SIDA, méningites ;
les maladies inflammatoires, notamment les maladies articulaires : arthrite, arthrite rhumatoïde, ostéoarthrite, spondylite, goutte, vascularite, maladie de Crohn, syndrome du colon irritable ;
l'ostéoporose ;
les affections oculaires : hypertension oculaire, glaucome ;
les affections pulmonaires : maladies des voies respiratoires, bronchospasmes, toux, asthme, bronchite chronique, obstruction chronique des voies respiratoires, emphysème ;
les maladies gastro-intestinales: syndrome du colon irritable, désordres inflammatoires intestinaux, ulcères, diarrhées ;
l'incontinence urinaire et l'inflammation vésicale.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvate dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvate ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvates.

## Revendications

1. Composé répondant à la formule (I) 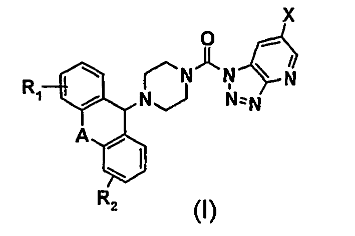 dans laquelle :
X représente un atome d'hydrogène, un atome d'halogène, un groupe (C₁-C₆)alkyle, (C₁-C₆)alcoxy, halo(C₁-C₆)alkyle, un groupe S(O)ₘR", hydroxy ou cyano,
A est absent ou bien représente une liaison, un atome d'oxygène, un atome de soufre, un groupe NR, C(O)NR', SO₂NR', un groupe (C₁-C₂)alkylène, ou un groupe (C₂)alcényle,
R₁ et R₂ représentent indépendamment l'un de l'autre, un ou plusieurs groupes choisis parmi un atome d'hydrogène, un groupe (C₁-C₆)alkyle, un groupe (C₁-C₆)alcoxy, (C₃-C₇)cycloalkyle, (C₃-C₇)cycloalkyle(C₁-C₆)alkyle, un groupe (C₃-C₇)cycloalkyle(C₁-C₆)alcoxy, un atome d'halogène, un groupe cyano, C(O)R', C(O)OR', C(O)NR₁₀R₂₀, NO₂, un groupe NR₁₀R₂₀, NR₁₀C(O)-R₂₀, les groupes (C₁-C₆)alkyle et (C₁-C₆)alcoxy étant éventuellement substitués par un ou plusieurs atomes ou groupes choisis indépendamment les uns des autres parmi les atomes d'halogène, les groupes hydroxy, amino, NR₁₀R₂₀,
R représente un groupe choisi parmi un atome d'hydrogène, un groupe (C₁-C₆)alkyle, (C₃-C₇)cycloalkyle, (C₃-C₇)cycloalkyle(C₁-C₆)alkyle, C(O)R', SO₂R", CO₂R", C(O)NR₁₀R₂₀,
R' représente un groupe choisi parmi un atome d'hydrogène, un groupe (C₁-C₆)alkyle, (C₃-C₇)cycloalkyle, (C₃-C₇)cycloalkyle(C₁-C₆)alkyle,
R" représente un groupe choisi parmi un groupe (C₁-C₆)alkyle, (C₃-C₇)cycloalkyle, (C₃-C₇)cycloalkyle(C₁-C₆)alkyle,
R₁₀ et R₂₀ représentent, indépendamment l'un de l'autre, un ou plusieurs groupes choisis parmi un atome d'hydrogène, un groupe (C₁-C₆)alkyle, (C₃-C₇)cycloalkyle, (C₃-C₇)cycloalkyle(C₁-C₆)alkyle ou bien R₁₀ et R₂₀ peuvent former un cycle saturé ou partiellement insaturé, contenant de 5 à 7 atomes de carbone et contenant éventuellement un hétéroatome choisi parmi O, N ou S(O)ₘ,
m représente 0, 1 ou 2,
à l'état de base ou de sel d'addition à un acide, ainsi qu'à l'état d'hydrate ou de solvate.

2. Composé de formule (I) selon la revendication 1, **caractérisé en ce que**
X représente un atome d'hydrogène ou d'halogène,
A est absent ou bien représente une liaison, un atome d'oxygène, un atome de soufre, un groupe NR, C(O)NR', SO₂NR', un groupe (C₁-C₂)alkylène, ou un groupe (C₂)alcényle,
à l'état de base ou de sel d'addition à un acide, ainsi qu'à l'état d'hydrate ou de solvate.

3. Composé de formule (I) selon l'une des revendications 1 ou 2, **caractérisé en ce que**
X représente un atome d'hydrogène ou d'halogène,
A est absent ou bien représente une liaison ou un groupe (C₁-C₂)alkylène,
R₁ et R₂ représentent indépendamment l'un de l'autre, un ou plusieurs groupes choisis parmi un atome d'hydrogène, un atome d'halogène, un groupe cyano ou alcoxy,
à l'état de base ou de sel d'addition à un acide, ainsi qu'à l'état d'hydrate ou de solvate.

4. Composé de formule (I) selon l'une des revendications 1 à 3, **caractérisé en ce que**
X représente un atome d'hydrogène ou d'halogène,
A est absent ou bien représente une liaison ou un groupe éthylène,
R₁ et R₂ représentent indépendamment l'un de l'autre un ou plusieurs groupes choisis parmi un atome d'hydrogène ou d'halogène,
à l'état de base ou de sel d'addition à un acide, ainsi qu'à l'état d'hydrate ou de solvate.

5. Composé de formule (I) selon l'une des revendications 1 à 4, **caractérisé en ce que**
X représente un atome d'hydrogène ou de chlore,
A est absent ou bien représente une liaison ou un groupe éthylène,
R₁ et R₂ représentent indépendamment l'un de l'autre un ou plusieurs groupes choisis parmi un atome d'hydrogène, un atome de fluor ou un atome de chlore, à l'état de base ou de sel d'addition à un acide, ainsi qu'à l'état d'hydrate ou de solvate.

6. Composé de formule (I) selon l'une des revendications 1 à 5, **caractérisé en ce qu'**il est choisi parmi :
- (4-benzhydryl-pipérazin-1-yl)-[1,2,3]triazolo[4,5-b]pyridin-1-yl-méthanone
- (4-benzhydryl-pipérazin-1-yl)-(6-chloro-[1,2,3]triazolo[4,5-b]pyridin-1-yl)-méthanone
- {4-[bis-(4-fluoro-phényl)-méthyl]-pipérazin-1-yl}-[1,2,3]triazolo[4,5-b]pyridin-1-yl-méthanone
- {4-[bis-(4-chloro-phényl)-méthyl]-pipérazin-1-yl}-[1,2,3]triazolo[4,5-b]pyridin-1-yl-méthanone
- {4-[bis-(4-fluoro-phényl)-méthyl]-pipérazin-1-yl}-(6-chloro-[1,2,3]triazolo[4,5-b]pyridin-1-yl)-méthanone
- {4-[bis-(4-chloro-phényl)-méthyl]-pipérazin-1-yl}-(6-chloro-[1,2,3]triazolo[4,5-b]pyridin-1-yl)-méthanone
- [4-(10,11-dihydro-5H-dibenzo[a,d]cycloheptèn-5-yl)-pipérazin-1-yl]-[1,2,3]triazolo[4,5-b]pyridin-1-yl-méthanone
- [4-(9H-fluorèn-9-yl)-pipérazin-1-yl]-[1,2,3]triazolo[4,5-b]pyridin-1-yl-méthanone.

7. Procédé de préparation d'un composé de formule (I) selon l'une quelconque des revendications 1 à 6, **caractérisé en ce que** l'on fait réagir un composé de formule (II) 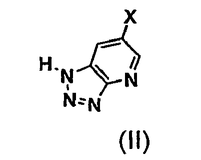 dans laquelle X est tel que défini dans la formule générale (I) selon la revendication 1, avec un composé de formule (III) 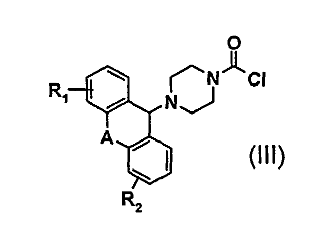 dans laquelle A, R₁ et R₂ sont tels que définis dans la formule générale (I) selon la revendication 1.

8. Procédé de préparation d'un composé de formule (I) selon l'une quelconque des revendications 1 à 6, **caractérisé en ce que** l'on fait réagir un composé de formule (IV) 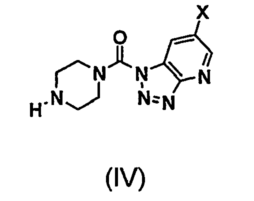 dans laquelle X est tel que défini dans la formule générale (I) selon la revendication 1, avec un composé de formule (V) 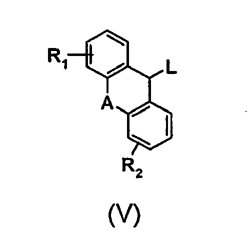 dans laquelle A, R₁ et R₂ sont tels que définis dans la formule générale (I) selon la revendication 1 et L représente un groupe partant.

9. Composé de formule (IV) 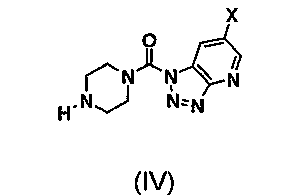 dans laquelle X est tel que défini dans la formule générale (I) selon la revendication 1.

10. Médicament, **caractérisé en ce qu'**il comprend un composé de formule (I) selon l'une quelconque des revendications 1 à 6, ou un sel d'addition de ce composé à un acide pharmaceutiquement acceptable, ou encore un hydrate ou un solvate du composé de formule (I).

11. Composition pharmaceutique, **caractérisée en ce qu'**elle comprend un composé de formule (I) selon l'une quelconque des revendications 1 à 6, ou un sel pharmaceutiquement acceptable, un hydrate ou un solvate de ce composé, ainsi qu'au moins un excipient pharmaceutiquement acceptable.

12. Utilisation d'un composé de formule (I) selon l'une quelconque des revendications 1 à 6 pour la préparation d'un médicament destiné au traitement et à la prévention d'une pathologie dans laquelle le 2-arachidonoylglycérol (2-AG) et le 1(3)-arachidonoylglycérol endogènes et/ou tout autre substrat métabolisé par l'enzyme MGL, sont impliqués.

13. Utilisation d'un composé de formule (I) selon l'une quelconque des revendications 1 à 6, à l'état de base, d'hydrate ou de solvate pharmaceutiquement acceptable, pour la préparation d'un médicament destiné à prévenir ou à traiter les douleurs aiguës ou chroniques, les vertiges, les vomissements, les nausées, les troubles du comportement alimentaire, le syndrome métabolique, les dyslipidémies, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires, virales ou bactériennes, les maladies inflammatoires, l'ostéoporose, les affections oculaires, les affections pulmonaires, les maladies gastro-intestinales, l'incontinence urinaire, l'inflammation vésicale.

14. Composé de formule (I) selon l'une quelconque des revendications 1 à 6, à l'état de base, d'hydrate ou de solvate pharmaceutiquement acceptable, pour la prévention ou le traitement des douleurs aiguës ou chroniques, des vertiges, des vomissements, des nausées, des troubles du comportement alimentaire, du syndrome métabolique, des dyslipidémies, des pathologies neurologiques et psychiatriques, des maladies neuro-dégénératives aiguës ou chroniques, de l'épilepsie, des troubles du sommeil, des maladies cardiovasculaires, de l'ischémie rénale, des cancers, des désordres du système immunitaire, des maladies allergiques, des maladies infectieuses parasitaires, virales ou bactériennes, des maladies inflammatoires, de l'ostéoporose, des affections oculaires, des affections pulmonaires, des maladies gastro-intestinales, de l'incontinence urinaire, de l'inflammation vésicale.

## Claims

1. Compound corresponding to formula (I) 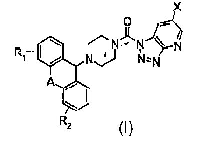 in which:
X is a hydrogen atom, a halogen atom, or a (C₁-C₆)alkyl, (C₁-C₆)alkoxy, halo(C₁-C₆)alkyl, S(O)ₘR'', hydroxyl or cyano group,
A is absent or else is a bond, an oxygen atom, a sulphur atom, an NR, C(C)NR' or SO₂NR' group, a (C₁-C₂)alkylene group or a (C₂)alkenyl group,
R₁ and R₂ are, independently of one another, one or more groups selected from a hydrogen atom, a (C₁-C₆)alkyl group, a (C₁-C₆)alkoxy group, a (C₃-C₇)cycloalkyl group, a (C₃-C₇)cycloalkyl(C₁-C₆)alkyl group, a (C₃-C₇)cycloalkyl(C₁-C₆)alkoxy group, a halogen atom, a cyano group, a C(O)R', C(O)OR', C(O)NR₁₀R₂₀ or NO₂ group, or an NR₁₀R₂₀ or NR₁₀C(O)-R₂₀ group, the (C₁-C₆)alkyl and (C₁-C₆)alkoxy groups being optionally substituted with one or more atoms or groups selected, independently of one another, from halogen atoms and hydroxyl, amino or NR₁₀R₂₀ groups,
R is a group selected from a hydrogen atom, and a (C₁-C₆) alkyl, (C₃-C₇)cycloalkyl, (C₃-C₇)cycloalkyl(C₁-C₆)alkyl, C(O)R', SO₂R'', CO₂R'' or C(O)NR₁₀R₂₀ group,
R' is a group selected from a hydrogen atom, a (C₁-C₆)alkyl group, a (C₃-C₇)cycloalkyl group and a (C₃-C₇)cycloalkyl(C₁-C₆)alkyl group,
R'' is a group selected from a (C₁-C₆)alkyl group, a (C₃-C₇)cycloalkyl group and a (C₃-C₇)cycloalkyl(C₁-C₆)alkyl group,
R₁₀ and R₂₀ are, independently of one another, one or more groups selected from a hydrogen atom, a (C₁-C₆)alkyl group, a (C₃-C₇)cycloalkyl group and a (C₃-C₇)cycloalkyl(C₁-C₆) alkyl group, or else R₁₀ and R₂₀ can form a saturated or partially unsaturated ring containing from 5 to 7 carbon atoms and optionally containing a heteroatom chosen from O, N or S(O)ₘ,
m represents 0, 1 or 2,
in the form of a base or of an addition salt with an acid, and also in the form of a hydrate or of a solvate.

2. Compound of formula (I) according to Claim 1, **characterized in that**:
X is a hydrogen or halogen atom,
A is absent or else is a bond, an oxygen atom, a sulphur atom, an NR, C(O)NR' or SO₂NR' group, a (C₁-C₂)alkylene group or a (C₂)alkenyl group,
in the form of a base or of an addition salt with an acid, and also in the form of a hydrate or of a solvate.

3. Compound of formula (I) according to either of Claims 1 and 2, **characterized in that**:
X is a hydrogen or halogen atom,
A is absent or else is a bond or a (C₁-C₂)alkylene group,
R₁ and R₂ are, independently of one another, one or more groups selected from a hydrogen atom, a halogen atom, a cyano group or an alkoxy group,
in the form of a base or of an addition salt with an acid, and also in the form of a hydrate or of a solvate.

4. Compound of formula (I) according to one of Claims 1 to 3, **characterized in that**:
X is a hydrogen or halogen atom,
A is absent or else is a bond or an ethylene group,
R₁ and R₂ are, independently of one another, one or more groups selected from a hydrogen or halogen atom,
in the form of a base or of an addition salt with an acid, and also in the form of a hydrate or of a solvate.

5. Compound of formula (I) according to one of Claims 1 to 4, **characterized in that**:
X is a hydrogen or chlorine atom,
A is absent or else is a bond or an ethylene group,
R₁ and R₂ are, independently of one another, one or more groups selected from a hydrogen atom, a fluorine atom or a chlorine atom,
in the form of a base or of an addition salt with an acid, and also in the form of a hydrate or of a solvate.

6. Compound of formula (I) according to one of Claims 1 to 5, **characterized in that** it is selected from:
- (4-benzhydrylpiperazin-1-yl)[1,2,3]triazolo[4,5-b]pyridin-1-ylmethanone
- (4-benzhydrylpiperazin-1-yl)(6-chloro[1,2,3]triazolo[4,5-b]pyridin-1-yl)methanone
- {4-[bis(4-fluorophenyl)methyl]piperazin-1-yl}[1,2,3]triazolo[4,5-b]pyridin-1-ylmethanone
- {4-[bis(4-chlorophenyl)methyl]piperazin-1-yl}[1,2,3]triazolo[4,5-b]pyridin-1-ylmethanone
- {4-[bis(4-fluorophenyl)methyl]piperazin-1-yl}(6-chloro[1,2,3]triazolo[4,5-b]pyridin-1-yl)methanone
- {4-[bis(4-chlorophenyl)methyl]piperazin-1-yl}(6-chloro[1,2,3]triazolo[4,5-b]pyridin-1-yl)methanone
- [4-(10,11-dihydro-5H-dibenzo[a,d]cyclohepten-5-yl)piperazin-1-yl][1,2,3]triazolo[4,5-b]pyridin-1-ylmethanone
- [4-(9H-fluoren-9-yl)piperazin-1-yl] [1,2,3]triazolo[4,5-b]pyridin-1-ylmethanone.

7. Process for preparing the compound of formula (I) according to any one of Claims 1 to 6, **characterized in that** a compound of formula (II) 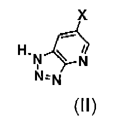 in which X is as defined in formula (I) according to Claim 1, is reacted with a compound of formula (III) 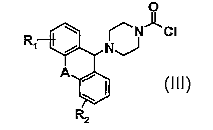 in which A, R₁ and R₂ are as defined in formula (I) according to Claim 1.

8. Process for preparing a compound of formula (I) according to any one of Claims 1 to 6, **characterized in that** a compound of formula (IV) 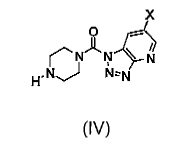 in which X is as defined in formula (I) according to Claim 1, is reacted with a compound of formula (V) 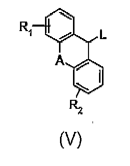 in which A, R₁ and R₂ are as defined in formula (I) according to Claim 1 and L represents a leaving group.

9. Compound of formula (IV) 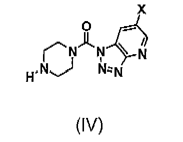 in which X is as defined in formula (I) according to Claim 1.

10. Medicament, **characterized in that** it comprises a compound of formula (I) according to any one of Claims 1 to 6, or an addition salt of this compound with a pharmaceutically acceptable acid, or else a hydrate or a solvate of the compound of formula (I).

11. Pharmaceutical composition, **characterized in that** it comprises a compound of formula (I) according to any one of Claims 1 to 6, or a pharmaceutically acceptable salt, a hydrate or a solvate of this compound, and also at least one pharmaceutically acceptable excipient.

12. Use of a compound of formula (I) according to any one of Claims 1 to 6, for the preparation of a medicament for use in the treatment and prevention of a pathological condition in which endogenous 2-arachidonoylglycerol (2-AG) and endogenous 1(3)-arachidonoylglycerol, and/or any other substrate metabolized by the MGL enzyme, are involved.

13. Use of a compound of formula (I) according to any one of Claims 1 to 6, in the form of a pharmaceutically acceptable base, hydrate or solvate, for the preparation of a medicament for use in preventing or treating acute or chronic pain, dizziness, vomiting, nausea, eating disorders, metabolic syndrome, dyslipidaemia, neurological and psychiatric pathological conditions, acute or chronic neurodegenerative diseases, epilepsy, sleep disorders, cardiovascular diseases, renal ischaemia, cancers, immune system disorders, allergic diseases, parasitic, viral or bacterial infectious diseases, inflammatory diseases, osteoporosis, ocular conditions, pulmonary conditions, gastrointestinal diseases, urinary incontinence and bladder inflammation.

14. Compound of formula (I) according to any one of Claims 1 to 6, in the form of a pharmaceutically acceptable base, hydrate or solvate, for the prevention or treatment of chronic or acute pain, vertigo, vomiting, nausea, eating disorders, metabolic syndrome, dyslipidaemia, neurological or psychiatric pathological conditions, acute or chronic neurodegenerative diseases, epilepsy, sleep disorders, cardiovascular diseases, renal ischaemia, cancers, immune system disorders, allergic diseases, parasitic, viral or bacterial infectious diseases, inflammatory diseases, osteoporosis, ocular conditions, pulmonary conditions, gastrointestinal diseases, urinary incontinence and bladder inflammation.

## Patentansprüche

1. Verbindung der Formel (I) 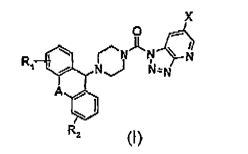 in der:
X ein für ein Wasserstoffatom, ein Halogenatom, eine (C₁-C₆)-Alkyl-, (C₁-C₆)-Alkoxy-, Halogen-(C₁-C₆)-alkylgruppe, eine S(O)ₘR"-Gruppe, Hydroxy oder Cyano steht,
A fehlt oder auch für eine Bindung, ein Sauerstoffatom, ein Schwefelatom, eine Gruppe NR, C(O)NR', SO₂NR', eine (C₁-C₂)-Alkylengruppe oder eine (C₂)-Alkenylgruppe steht,
R₁ und R₂ unabhängig voneinander für eine oder mehrere Gruppen stehen, die aus einem Wasserstoffatom, einer (C₁-C₆)-Alkylgruppe, einer (C₁-C₆)-Alkoxygruppe, einer (C₃-C₇)-Cycloalkyl-, (C₃-C₇)-Cycloalkyl-(C₁-C₆)-alkyl-, (C₃-C₇)-Cycloalkyl-(C₁-C₆)-alkoxygruppe, einem Halogenatom, einer Cyanogruppe, C(O)R', C(O)OR', C(O)NR₁₀R₂₀, NO₂, einer NR₁₀R₂₀-Gruppe, NR₁₀C(O)-R₂₀, den (C₁-C₆)-Alkylgruppen und (C₁-C₆)-Alkoxygruppen ausgewählt sind, die gegebenenfalls durch ein oder mehrere Atome oder Gruppen substituiert sind, die unabhängig voneinander aus den Halogenatomen, den Hydroxy-, Amino-, NR₁₀R₂₀-Gruppen ausgewählt sind,
R für eine Gruppe steht, die aus einem Wasserstoffatom, einer (C₁-C₆)-Alkyl-, (C₃-C₇)-Cycloalkyl-, (C₃-C₇)-Cycloalkyl-(C₁-C₆)-alkyl-, C(O)R'-, SO₂R"-, CO₂R"- oder C(O)NR₁₀R₂₀-Gruppe ausgewählt ist,
R' für eine Gruppe steht, die aus einem Wasserstoffatom, einer (C₁-C₆)-Alkyl-, (C₃-C₇)-Cycloalkyl-, (C₃-C₇)-Cycloalkyl-(C₁-C₆)-alkylgruppe ausgewählt ist, R" für eine Gruppe steht, die aus einer (C₁-C₆)-Alkyl-, (C₃-C₇)-Cycloalkyl- oder (C₃-C₇)-Cycloalkyl-(C₁-C₅)-alkylgruppe ausgewählt ist,
R₁₀ und R₂₀ unabhängig voneinander für eine oder mehrere Gruppen stehen, die aus einem Wasserstoffatom, einer (C₁-C₆)-Alkyl-, (C₃-C₇)-Cycloalkyl- oder (C₃-C₇)-Cycloalkyl-(C₁-C₆)-alkylgruppe ausgewählt sind, oder R₁₀ und R₂₀ einen gesättigten oder teilweise ungesättigten Ring mit 5 bis 7 Kohlenstoffatomen bilden können, der gegebenenfalls ein Heteroatom, ausgewählt aus O, N oder S(O)ₘ, enthält,
m für 0, 1 oder 2 steht,
im basischen Zustand oder als Säureadditionssalz, sowie im Hydrat- oder Solvatzustand.

2. Verbindung der Formel (I) nach Anspruch 1, **dadurch gekennzeichnet, dass**
X für ein Wasserstoff- oder Halogenatom steht,
A fehlt oder auch für eine Bildung, ein Sauerstoffatom, ein Schwefelatom, eine Gruppe NR, C(O)NR', SO₂NR', eine (C₁-C₂)-Alkylengruppe oder eine (C₂)-Alkenylgruppe steht, im basischen Zustand oder als Säureadditionssalz, sowie im Hydrat- oder Solvatzustand.

3. Verbindung der Formel (I) nach einem der Ansprüche 1 oder 2, **dadurch gekennzeichnet, dass**
X für ein Wasserstoff- oder Halogenatom steht,
A fehlt oder auch für eine Bindung oder eine (C₁-C₂)-Alkylengruppe steht,
R₁ und R₂ unabhängig voneinander für eine oder mehrere Gruppen stehen, die aus einem Wasserstoffatom, einem Halogenatom, einer Cyano- oder Alkoxygruppe ausgewählt sind,
im basischen Zustand oder als Säureadditionssalz, sowie im Hydrat- oder Solvatzustand.

4. Verbindung der Formel (I) nach einem der Ansprüche 1 bis 3, **dadurch gekennzeichnet, dass**
X für ein Wasserstoff- oder Halogenatom steht,
A fehlt oder auch für eine Bindung oder eine Ethylengruppe steht,
R₁ und R₂ unabhängig voneinander für eine oder mehrere Gruppen stehen, die aus einem Wasserstoff- oder Halogenatom ausgewählt sind,
im basischen Zustand oder als Säureadditionssalz, sowie im Hydrat- oder Solvatzustand.

5. Verbindung der Formel (I) nach einem der Ansprüche 1 bis 4, **dadurch gekennzeichnet, dass**
X für ein Wasserstoff- oder Chloratom steht,
A fehlt oder auch für eine Bindung oder eine Ethylengruppe steht,
R₁ und R₂ unabhängig voneinander für eine oder mehrere Gruppen stehen, die aus einem Wasserstoffatom, einem Fluoratom oder einem Chloratom ausgewählt sind,
im basischen Zustand oder als Säureadditionssalz, sowie im Hydrat- oder Solvatzustand.

6. Verbindung der Formel (I) nach einem der Ansprüche 1 bis 5, **dadurch gekennzeichnet, dass** sie ausgewählt wird aus:
(4-Benzhydrylpiperazin-1-yl)-[1,2,3]triazolo[4,5-b]pyri din-1-ylmethanon
(4-Benzhydrylpiperazin-1-yl)-(6-chlor[1,2,3]triazolo[4, 5-b]pyridin-1-yl)methanon
{4-[Bis-(4-fluorphenyl)methyl]piperazin-1-yl}-[1,2,3]tr iazolo[4,5-b]pyridin-1-ylmethanon
{4-[Bis-(4-chlorphenyl)methyl]piperazin-1-yl}-[1,2,3] triazolo[4,5-b]pyridin-1-ylmethanon
{4-[Bis-(4-fluorphenyl)methyl]piperazin-1-yl}-(6-chlor[ 1,2,3]triazolo[4,5-b]pyridin-1-yl)methanon
{4-[Bis-(4-chlorphenyl)methyl]piperazin-1-yl}-(6-chlor[ 1,2,3]triazolo[4,5-b]pyridin-1-yl)methanon
[4-(10,11-Dihydro-5H-dibenzo[a,d]cyclohepten-5-yl)piper azin-1-yl]-[1,2,3]triazolo[4,5-b]pyridin-1-ylmethanon
[4-(9H-Fluoren-9-yl)piperazin-1-yl]-[1,2,3]triazolo[4,5 -b]pyridin-1-ylmethanon.

7. Verfahren zur Herstellung einer Verbindung der Formel (I) nach einem der Ansprüche 1 bis 6, **dadurch gekennzeichnet, dass** eine Verbindung der Formel (II) 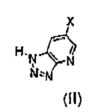 in der X wie in der allgemeinen Formel (I) nach Anspruch 1 definiert ist, mit einer Verbindung der Formel (III) 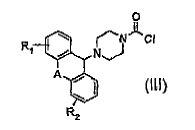 in der A, R₁ und R₂ wie in der allgemeinen Formel (I) nach Anspruch 1 definiert sind, zur Reaktion gebracht wird.

8. Verfahren zur Herstellung einer Verbindung der Formel (I) nach einem der Ansprüche 1 bis 6, **dadurch gekennzeichnet, dass** eine Verbindung der Formel (IV) 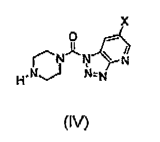 in der X wie in der allgemeinen Formel (I) nach Anspruch 1 definiert ist, mit einer Verbindung der Formel (V) 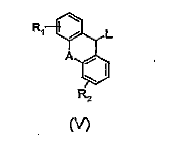 in der A, R₁ und R₂ wie in der allgemeinen Formel (I) nach Anspruch 1 definiert sind und L für eine Abgangsgruppe steht, zur Reaktion gebracht wird.

9. Verbindung der Formel (IV) 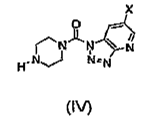 in der X wie in der allgemeinen Formel (I) nach Anspruch 1 definiert ist.

10. Arzneimittel, **dadurch gekennzeichnet, dass** es eine Verbindung der Formel (I) nach einem der vorhergehenden Ansprüche 1 bis 6 oder ein Additionssalz dieser Verbindung mit einer pharmazeutisch unbedenklichen Säure oder auch ein Hydrat oder ein Solvat der Verbindung der Formel (I) umfasst.

11. Pharmazeutische Zusammensetzung, **dadurch gekennzeichnet, dass** sie eine Verbindung der Formel (I) nach einem der vorhergehenden Ansprüche 1 bis 6 oder ein pharmazeutisch unbedenkliches Salz, ein Hydrat oder ein Solvat dieser Verbindung sowie mindestens ein pharmazeutisch unbedenkliches Hilfsmittel umfasst.

12. Verwendung einer Verbindung der Formel (I) nach einem der Ansprüche 1 bis 6 zur Herstellung eines Arzneimittels, das zur Behandlung und der Vorbeugung einer Pathologie bestimmt ist, bei der endogenes 2-Arachidonoylglycerin (2-AG) und 1(3)-Arachidonoylglycerin und/oder jedes andere durch das Enzym MGL metabolisierte Substrat betroffen ist.

13. Verwendung einer Verbindung der Formel (I) nach einem der Ansprüche 1 bis 6 im basischen Zustand, als pharmazeutisch unbedenkliches Hydrat oder Solvat zur Herstellung eines Arzneimittels, das zur Vorbeugung oder zur Behandlung von akuten oder chronischen Schmerzen, Schwindel, Erbrechen, Übelkeit, Störungen des Ernährungsverhaltens, des metabolischen Syndroms, Dyslipidämien, neurologischen oder psychiatrischen Pathologien, akuten oder chronischen neurodegenerativen Erkrankungen, Epilepsie, Schlafstörungen, kardiovaskulären Erkrankungen, renaler Ischämie, Karzinomen, Störungen des Immunsystems, allergischen Erkrankungen, parasitären, viralen oder bakteriellen Infektionskrankheiten, entzündlichen Krankheiten, Osteoporose, Augenleiden, Lungenerkrankungen, gastrointestinalen Erkrankungen, Urininkontinenz oder Blasenentzündungen bestimmt ist.

14. Verbindung der Formel (I) nach einem der Ansprüche 1 bis 6 im basischen zustand, als pharmazeutisch unbedenkliches Hydrat oder Solvat zur Vorbeugung oder zur Behandlung von akuten oder chronischen Schmerzen, Schwindel, Erbrechen, Übelkeit, Störungen des Ernährungsverhaltens, des metabolischen Syndroms, Dyslipidämien, neurologischen oder psychiatrischen Pathologien, akuten oder chronischen neurodegenerativen Erkrankungen, Epilepsie, Schlafstörungen, kardiovaskulären Erkrankungen, renaler Ischämie, Karzinomen, Störungen des Immunsystems, allergischen Erkrankungen, parasitären, viralen oder bakteriellen Infektionskrankheiten, entzündlichen Krankheiten, Osteoporose, Augenleiden, Lungenerkrankungen, gastrointestinalen Erkrankungen, Urininkontinenz oder Blasenentzündungen bestimmt ist.
